Europäisches Patentamt

European Patent Office

Office européen des brevets

(11) EP 0 993 820 A1

(19)

(12) # DEMANDE DE BREVET EUROPEEN

(43) Date de publication:
19.04.2000 Bulletin 2000/16

(51) Int Cl.⁷: **A61K 7/027**, A61K 7/032

(21) Numéro de dépôt: **99401799.4**

(22) Date de dépôt: **16.07.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **14.09.1998 FR 9811433**

(71) Demandeur: **L'OREAL**
**75008 Paris (FR)**

(72) Inventeurs:
• **Ferrari, Véronique**
**94700 Maisons-Alfort (FR)**
• **de la Poterie, Valérie**
**77820 Le Chatelet-en-Brie (FR)**

(74) Mandataire: **Lhoste, Catherine**
**L'OREAL-DPI**
**6 rue Bertrand Sincholle**
**92585 Clichy Cédex (FR)**

(54) **Composition cosmétique aqueuse comprenant un polyester sulfoné et un composé siliconé**

(57)     L'invention a pour objet une composition cosmétique ou dermatologique comprenant dans une phase aqueuse:

i) un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \qquad (I)$$

dans laquelle

- A représente un groupement 1,4-phénylène, sulfo-1,3-phénylène,
- n va de 1 à 4,

- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,

la masse moléculaire en poids dudit oligomère copolyester étant inférieure à 20 000,
ii) au moins un composé siliconé en émulsion dans la phase aqueuse.

Cette composition est destinée au maquillage des matières kératiniques.

## Description

La présente invention concerne une nouvelle composition filmogène sans transfert et résistant à l'eau à application topique, comprenant un agent gélifiant hydrophile particulier de type polyester sulfoné et un composé siliconé, destinée en particulier aux domaines cosmétique et/ou dermatologique. L'invention se rapporte aussi à l'utilisation de cette composition pour le maquillage ou le traitement cosmétique des matières kératiniques d'être humain telles que la peau, les ongles, les cils, les sourcils, les cheveux, ou des muqueuses telles que les lèvres. Cette composition peut se présenter sous forme de gel, de pâte ou de produit coulé, notamment de stick.

Les produits de maquillage ou de soin de la peau ou des lèvres comme les fonds de teints ou les rouges à lèvres contiennent généralement des phases grasses telles que des cires et des huiles, des pigments et/ou des charges et, éventuellement des additifs comme des actifs cosmétiques ou dermatologiques. Elles peuvent contenir des gélifiants permettant d'obtenir la consistance souhaitée pour la composition selon son application.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. En outre, l'apparition de ces traces inacceptables notamment sur les cols de chemisier peut écarter certaines femmes de l'utilisation de ce type de maquillage.

Il est connu des demandes EP-A-602905 et JP-A-6165809 des compositions de maquillage dites "sans transfert" comprenant des huiles volatiles, notamment des huiles de silicones volatiles et/ou des huiles hydrocarbonées volatiles. Toutefois, ces compositions sont souvent inconfortables à porter (sensation de tiraillement et de dessèchement) notamment sur les lèvres et conduisent à un maquillage mat.

Il a également été proposé dans la demande EP-A-775483 des compositions pour les lèvres contenant une dispersion aqueuse de polymère. Bien que ces compositions conduisent à des films ayant de bonnes propriétés sans transfert, ces films ont malheureusement l'inconvénient d'être inconfortables au cours du temps. De plus, ces films sont souvent difficiles à démaquiller avec les démaquillants habituellement utilisés.

Le but de la présente invention est de proposer une composition filmogène aqueuse ne présentant pas les inconvénients évoqués ci-dessus et présentant notamment des propriétés de sans transfert total, de brillance, d'absence de tiraillement pendant et après l'application sur les matières kératiniques, et facile à démaquiller.

Les inventeurs ont découvert qu'une telle composition pouvait être obtenue en utilisant un agent gélifiant hydrophile particulier de type polyester sulfoné et un composé siliconé en émulsion aqueuse. On obtient alors une composition laissant après l'application un film présentant une bonne brillance et une bonne tenue, ne transférant pas du tout. Le film est notamment non collant et confortable (non tiraillement) pendant et après l'application de la composition sur les matières kératiniques. Le film produit également une sensation de fraîcheur et est facile à démaquiller avec les démaquillants habituellement utilisés. De plus, la composition s'applique facilement sur les matières kératiniques.

Cependant, il est connu dans la demande FR-A-2760642, publiée le 18/09/1998, une composition filmogène contenant un oligomère copolyester téréphtalique sulfoné et 2 % en poids d'un aminosilicone en émulsion aqueuse.

La présente invention a donc pour objet une nouvelle composition filmogène à application topique comprenant dans une phase aqueuse un agent gélifiant hydrophile de type polyester sulfoné, caractérisée par le fait que l'agent gélifiant hydrophile est un oligomère copolyester téréphtalique hydrosoluble ou hydrodispersible et que la composition comprend également au moins un composé siliconé en émulsion dans la phase aqueuse.

Les agents gélifiants particuliers utilisés selon l'invention sont des oligomères copolyesters téréphtaliques hydrosolubles ou hydrodispersables comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs pour lesquels A représente un groupement 1,4-phénylène et n est égal à 1,
- au moins 7 % en mole desdits motifs de formule (I) étant des motifs pour lesquels A représente un groupement sulfo-1,3-phénylène,

la masse moléculaire en poids desdits oligomères copolyesters étant inférieure à 20 000, de préférence inférieure à 15 000.

De manière préférentielle, au moins 40 % en mole, et plus préférentiellement entre 40 et 90 % en mole des motifs de formule (I) sont des motifs pour lesquels A représente un groupement 1,4-phénylène et n est égal à 1.

De préférence, au moins 10 % en mole, plus préférentiellement entre 10 % et 25 % en mole des motifs de formule (I) sont des motifs pour lesquels A représente un groupement sulfo-1,3-phénylène.

Les extrémités des chaînes desdits oligomères copolyesters peuvent être semblables ou différentes et représentées essentiellement par les groupements de formule (I'):

$$-CO\text{-}A\text{-}CO\text{-}O\text{-}(CH_2\text{-}CH_2\text{-}O)_n\text{-}H \qquad\qquad (I')$$

dans laquelle A et n sont définis ci-dessus.

Lesdits oligomères peuvent également présenter en extrêmités de chaîne, et en quantité mineure, des groupements de formules

$$-A\text{-}CO\text{-}OH$$

$$-A\text{-}CO\text{-}OR$$

formules dans lesquelles A est défini ci-dessus et R représente un groupement alkyle en $C_1$-$C_4$.

Lorsque A représente un groupement sulfo-1,3-phénylène, il s'agit plus particulièrement d'un sulfonate de métaux alcalins, notamment de sodium ou de potassium, ou d'un sulfonate d'ammonium ou de mono-, di-, tri- ou tétra-alkylammonium inférieur. Par alkylammonium inférieur, on entend de préférence un ammonium dont le ou les radicaux alkyles sont des alkyles inférieurs, de préférence en $C_1$-$C_6$. De manière préférentielle, il s'agit d'un sulfonate de sodium.

L'oligomère copolyester peut comprendre éventuellement jusqu'à 20 % en mole, de préférence de 0,5 à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids allant de 5 000 et 14 000, et plus préférentiellement de 8 000 à 10 000.

Les masses moléculaires en poids sont mesurées par chromatographie par perméation de gel dans le diméthylacétamide contenant $10^{-2}$ N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions

- . d'estérification de diacides et de diols et polycondensation
- . de transestérification de diesters et de diols et polycondensation
- . d'autocondensation d'hydroxyacides
- . de Schotten-Baumann par mise en oeuvre de diols et de chlorures d'acide et polycondensation
- . de polymérisation de lactones

en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les oligomères copolyesters téréphtaliques utilisés selon l'invention peuvent être préparés par estérification et/ou transestérification / polycondensation d'une composition monomère à base:

- d'acide, anhydride ou diester téréphtalique (Tp)
- d'acide, anhydride ou diester sulfoisophtalique (Slp)
- éventuellement d'acide, anhydride ou diester isophtalique (Ip)
- et d'éthylène glycol (EG)

selon des quantités relatives correspondant à

* un rapport molaire (Slp)/[(Tp)+(Slp)+(Ip)] d'au moins 7/100, de préférence d'au moins 10/100, tout particulièrement de 10/100 à 25/100
* un rapport molaire (Ip)/[(Tp)+(Slp)+(Ip)] de 20/100 au plus, de préférence de 5/100 au plus
* un rapport molaire (EG) / [(Tp)+(Slp)+(Ip)] de 2/1 à 3/1.

en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en $C_1$-$C_4$), de diméthyle de préférence.

Le monomère sulfoisophtalique (Slp) est de préférence mis en oeuvre sous la forme d'un sulfonate de métal alcalin (sodium notamment) de diester inférieur (d'alkyle en $C_1$-$C_4$), de méthyle de préférence. On peut citer tout particulièrement le sodio-oxysulfonyl-5 isophtalate de diméthyle.

Le monomère isophtalique (Ip) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation. Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2"-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001% à 0,05% par rapport au poids de réactifs présents.

La durée de l'opération d'interéchange est de 1 à 4 heures ; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification. L'opération est réalisée en présence des mêmes types de catalyseur et de limiteur d'éthérification que ceux mis en oeuvre lors de l'opération de transestérification, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997.

L'oligomère copolyester téréphtalique peut être présent dans la composition selon l'invention en une teneur allant de 0,5 % à 40 % en poids, par rapport au poids total de la composition. Selon la forme recherchée, gélifiée, pâteuse ou solide, on emploiera des quantités différentes de gélifiant particulier. Pour une composition solide, on emploiera de préférence de 10 à 40 % en poids de gélifiant particulier, plus préférentiellement de 15 à 30 % en poids. Pour une composition gélifiée ou pâteuse, on emploiera de préférence de 0,5 à 10 % en poids de gélifiant particulier, plus préférentiellement de 2 à 5 % en poids.

Le composé siliconé présent dans la composition selon l'invention se trouve sous la forme d'émulsion aqueuse, appelée encore émulsion de silicone.

Par émulsion aqueuse, on entend une émulsion de type huile-dans-eau dans laquelle le composé siliconé est dispersé sous forme de particules ou de gouttelettes dans la phase aqueuse formant la phase continue de l'émulsion. Cette émulsion peut être stabilisée par un système émulsionnant usuel.

Cette émulsion de silicone peut avoir une taille de gouttelettes ou de particules de silicone allant de 10 nm à 50 $\mu$m, et de préférence de 10 nm à 1000 nm. Elle peut également se présenter sous forme de microémulsion, ayant une taille de gouttelettes de silicone de l'ordre de 10 à 80 nm. Les microémulsions de silicone sont des émulsions stables de particules colloïdales et sont généralement transparentes.

Les composés siliconés en émulsion sont de préférence des polyorganosiloxanes, qui peuvent se présenter sous forme d'huiles, notamment d'huile de silicone volatile ou non volatile, de gommes, de résines, de produits pâteux, ou de cires, ou leurs mélanges.

Ces composés siliconés sont généralement facilement émulsifiables dans un milieu aqueux à l'aide de tensioactifs employés habituellement avec ces composés siliconés, comme les (alkyl)diméthicones copolyols ou les tensioactifs cités dans le document EP-A-678015.

Les gommes, cires et résines de silicone peuvent être mélangées avec des huiles de silicones dans lesquelles elles peuvent être solubilisées, le mélange étant sous la forme d'émulsion dans la phase aqueuse de la composition selon l'invention.

D'une façon générale, les composés siliconés sont de préférence des polymères contenant des motifs répétitifs de formule (II) : $R_nSiO_{(4-n)/2}$

Les substituants R présents dans ces motifs répétitifs sont des groupements organiques, et peuvent être identiques ou différents. En outre, un même composé peut contenir des motifs répétitifs différents.

Les motifs répétitifs correspondant à n = 2 correspondent à un composé ayant une structure linéaire ou cyclique dont la chaîne est constituée de liaisons siloxane. Dans le cas d'un polymère linéaire, des motifs correspondant à n = 3 constituent les groupements terminaux.

En outre, les composés siliconés peuvent contenir des motifs réticulants intercalés entre les motifs répétitifs. Ces motifs réticulants correspondent à la formule (II) ci-dessus avec n = 1 ou n = 0.

Les motifs répétitifs pour lesquels n = 4 corespondent à un polymère réticulé dont les groupements terminaux peuvent être des motifs pour lesquels n = 1 ; 2 ou 3.

Les groupements R de la formule (II) peuvent représenter notamment des groupements alkyle, alcényle, cycloalkyle, aryle, alkylaryle ou hydroxyle, et peuvent comporter en outre, des groupements fonctionnels tels qu'éthers, amines, carboxyles, hydroxyles, thiols, esters, sulfonates, sulfates.

Les groupements alkyle ont par exemple 1 à 50 atomes de carbone ; les groupements cycloalkyle ont par exemple 5 ou 6 atomes de carbone ; les groupements aryle sont notamment des groupements phényle ; les groupements alcényle ont notamment de 2 à 10 atomes de carbone ; les groupements alkylaryle peuvent avoir de 7 à 20 atomes de carbone.

Dans le cas des groupements terminaux correspondant à n = 3, l'un des groupements R attachés au silicium terminal peut en outre représenter un autre groupement, tel qu'un groupement OH.

Parmi les composés siliconés susceptibles d'être utilisés dans la composition de la présente invention, on peut citer les silicones non volatiles, choisies en particulier parmi les polyorganosiloxanes et notamment les polyalkylsiloxanes, les polyarylsiloxanes, les polyalkylarylsiloxanes, les copolymères polyéthersiloxanes, organomodifiés ou non, les gommes, les produits pâteux, les cires et les résines de silicones, les polysiloxanes modifiés par des groupements organofonctionnels ainsi que leurs mélanges.

Ils sont choisis plus particulièrement parmi les polyalkylsiloxanes, parmi lesquels on peut citer principalement les polydiméthylsiloxanes linéaires à groupements terminaux triméthylsilyle ayant une viscosité de $10^{-5}$ à 2,5 m²/s à 25°C, de préférence de $1.10^{-5}$ à 1 m²/s à 25°C, et notamment les produits commerciaux suivants:

- les huiles SILBIONE des séries 47 et 70047 commercialisées par RHONE POULENC telles que par exemple l'huile 47V500000,

- les huiles de la série 200 de DOW CORNING,
- les huiles VISCASIL de GENERAL ELECTRIC,
- certaines huiles des séries SF (SF96, SF18) de GENERAL ELECTRIC.

On peut également citer les polydiméthylsiloxanes linéaires à groupements terminaux diméthylsilanol tels que les huiles de la série 48 de RHONE POULENC.

On peut également citer les produits vendus sous les dénominations ABIL WAX 9800 et 9801 par la société GOLDSCHMIDT qui sont des polyalkyl ($C_1$-$C_{20}$)-siloxanes.

Parmi les polyalkylarylsiloxanes, on peut citer les polydiméthylméthylphénylsiloxanes et les polydiméthyldiphénylsiloxanes, linéaires ou ramifiés, ayant une viscosité de $1.10^{-5}$ à $5.10^{-2}$ $m^2$/s à 25°C, et notamment les produits commerciaux suivants :

- les huiles SILBIONE de la série 70641 ou 70633 V 30 de RHONE POULENC,
- les huiles des séries RHODORSIL 70633 et 763 de RHONE POULENC,
- l'huile DC 556 Fluid ou SF 558 de DOW CORNING,
- les silicones de la série PK de BAYER comme le produit PK20,
- les silicones des séries PN et PH de BAYER comme les produits PN1000 et PH1000,
- certaines huiles des séries SF de GENERAL ELECTRIC telles que SF1023, SF1154, SF1250 et SF1265,
- les huiles ABIL AV 8853 de GOLDSCHMIDT.

Les gommes de silicone utilisables conformément à la présente invention, sont notamment des polydiorganosiloxanes ayant des masses moléculaires élevées, de préférence comprises entre 200.000 et 1.000.000. Elles peuvent être utilisées seules ou en mélange dans un solvant qui peut être choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On peut citer plus particulièrement les produits suivants : les gommes polydiméthylsiloxane/méthylvinylsiloxane, polydiméthylsiloxane/diphénylsiloxane, polydiméthylsiloxane/phénylméthylsiloxane, polydiméthylsiloxane/diphénylsiloxane/ méthylvinylsiloxane.

On peut notamment employer des mélanges tels que :

- les mélanges formés à partir d'un polydiméthylsiloxane hydroxylé en bout de chaîne (diméthiconol selon le CTFA) et d'un polydiméthylsiloxane cyclique (cyclométhicone selon le CTFA) tel que le produit Q2-1401 vendu par la société DOW CORNING,
- les mélanges formés à partir d'une gomme polydiméthylsiloxane avec une huile de silicone cyclique tel que le produit SF1214 de GENERAL ELECTRIC (qui est un mélange de gomme diméthicone ayant un poids moléculaire de 500.000 solubilisée dans le décaméthylcyclopentasiloxane).
- les mélanges de deux PDMS de viscosités différentes, notamment d'une gomme PDMS et d'une huile PDMS, tels que le produit SF1236 de la société GENERAL ELECTRIC (qui est un mélange de 15% de gomme diméthicone ayant un poids moléculaire de 500.000, d'une viscosité de $20 m^2$/s et de 85% d'huile SF96 d'une viscosité de $5.10^{-6}$ $m^2$/s).

Comme cires de silicone, on peut citer celles décrites dans les documents FR-A-2688134 et EP-A-811372.

Les résines d'organopolysiloxanes utilisables selon l'invention sont notamment des systèmes siloxaniques réticulés renfermant les unités $R_2SiO$, $RSiO_{3/2}$ et $SiO_2$. Parmi ces composés, les produits plus particulièrement préférés sont ceux dans lesquels R désigne un alkyle en C1-C36 ou phényle.

Parmi ces résines, on peut citer le produit vendu sous la dénomination DOW CORNING 593 ou ceux vendus sous les dénominations Silicone Fluid SS4230 et SS4267 par la société GENERAL ELECTRIC et qui sont de type diméthyl/ triméthyl siloxane.

Les silicones organomodifiées sont des composés siliconés tels que définis ci-dessus, comportant en outre dans leur structure un ou plusieurs groupements organofonctionnels directement fixés sur la chaîne siloxanique ou fixés par l'intermédiaire d'un radical hydrocarboné.

On peut citer, par exemple, les silicones comportant :

- des groupements polyéthylèneoxy et/ou polypropylèneoxy comportant éventuellement des groupes alkyles tels que :

  - les diméthicone copolyols et notamment celui vendu par la société DOW CORNING sous la dénomination DC1248,

- les alkyl(di)méthicone copolyols et notamment l'alkyl ($C_{12}$) méthicone copolyol vendu par la société DOW CORNING sous la dénomination Q2-5200.
- les huiles SILWET L 722, L 7500, L 77, L 711 de la société UNION CARBIDE.

- des groupements aminés, éventuellement substitués, comme les produits vendus sous la dénomination GP4 Silicone Fluid et GP7100 par la société GENESEE ou les produits vendus sous les dénominations Q2-8220 et DC929 par la société DOW CORNING. Les groupements aminés substitués sont en particulier des groupements aminoalkyle en $C_1$-$C_4$.
- des groupements thiols comme dans les GP 72 A et GP 71 de GENESEE. - des groupements carboxylates comme dans les produits décrits dans le brevet EP 186 507 de la société CHISSO CORPORATION.
- des groupements alkoxylés, comme les produits vendus sous la dénomination Silicone copolymer F-755 par SWS SILICONES, ou ABIL WAX 2428, 2434 et 2440 par la société GOLDSCHMIDT.
- des groupements hydroxylés, comme les polyorganosiloxanes à fonction hydroxyalkyle notamment décrits dans la demande de brevet français 85FR-163 34.
- des groupements acyloxyalkyle, comme par exemple les polyorganosiloxanes décrits dans la demande de brevet français 88FR-17433. Ces composés peuvent être préparés par estérification de polyorganosiloxanes à fonction hydroxyalkyle.
- des groupements anioniques de type carboxylique tels que les groupements alkylcarboxyliques; 2-hydroxy alkylsulfonate ou 2-hydroxyalkylthiosulfate,
- des groupements fluorés.

Parmi les composés siliconés susceptibles d'être utilisés dans la composition selon l'invention, on peut encore citer les silicones volatiles, qui possèdent généralement un point d'ébullition compris entre 60 °C et 260°C et une viscosité inférieure à $10^{-5}$ m$^2$/s, et en particulier :

- les silicones cycliques comportant de 3 à 7 atomes de silicium et de préférence 4 à 6 atomes de silicium tels que l'octaméthylcyclotétrasiloxane, le décaméthylcyclopentasiloxane et leurs mélanges.

On peut également citer les cyclocopolymères tels que le diméthylsiloxane/méthylalkylsiloxane, et notamment la silicone volatile FZ 3109 vendue par la société UNION CARBIDE.
On peut encore citer les mélanges de silicones cycliques avec des composés dérivés du silicium tels que le mélange d'octaméthylcyclotétrasiloxane et de tétra triméthylsilyl pentaérythritol (50/50) et le mélange d'octaméthylcyclotétrasiloxane et d'oxy-1,1'(hexa-2,2,2',2',3,3' triméthylsilyloxy) bis-néopentane.

- les silicones volatiles linéaires ayant 2 à 10 atomes de carbone et une viscosité inférieure ou égale à $10^{-5}$ m$^2$/s à 25°C, comme les polydiméthylsiloxane (PDMS) tels que l'hexaméthyldisiloxane, le décaméthyltétrasiloxane, ou le dodécaméthylcyclohexasiloxane et celles citées dans le document EP-A-811372. Des silicones entrant dans cette classe sont également décrits dans l'article publié dans Cosmetic and Toiletries, Vol 91, Jan 76, p 27-32 intitulé "Volatile Silicone fluids for cosmetics".

L'émulsion aqueuse de composés siliconés pourra aisément être préparée par l'homme du métier, sur base de ses connaissances générales.
Parmi les émulsions aqueuses de silicone commercialement disponibles, on peut citer :

- les émulsions de polydiméthylsiloxane (SM2162 de General Electric),
- les émulsions de stéaryldiméthicone (SLM23032 de Wacker),
- les microémulsions d'amodiméthicone (Microémulsion 71827 de Rhône-Poulenc),
- les microémulsions cationiques de polydiméthylsiloxane à groupements aminoéthylaminopropyl (DC939 de Dow Corning),
- les microémulsions de polydiméthylsiloxane (SILTECH MFF 5015-70 de Siltech ou DC2-1281 de Dow Corning)
- les émulsions cationiques de polydiméthylsiloxanes aminés (BELSIL ADM 6057 E de Wacker)
- les émulsions de polydiméthylsiloxanes phénylés (MIRASIL DPDM-E de Rhône Poulenc)
- les émulsions de polydiméthylsiloxane alkylés (SILRES M50E de Wacker).

Les composés siliconés peuvent être présents dans la composition à raison de 0,1% à 30% en poids de matière sèche de composé siliconé, par rapport au poids total de la composition, et de préférence à raison de 2% à 10% en poids.
En particulier, la composition selon l'invention ne contient pas 2 % en poids, par rapport au poids total de la composition, d'aminosilicone.

Par ailleurs, la composition selon l'invention peut contenir des adjuvants couramment utilisés dans les compositions cosmétiques, notamment topiques. On peut citer à titre d'exemple d'adjuvants les colorants, les pigments, les nacres, les agents anti-UV, les conservateurs, les agents épaississants, les agents plastifiants, les tensioactifs, les cires, les huiles, les parfums, les agents modificateurs de pH, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être utilisée pour le maquillage, le soin ou le traitement cosmétique non thérapeutique des matières kératiniques et/ou des muqueuses. La composition de maquillage peut être un produit de maquillage des lèvres, notamment sous forme de pâte ou de stick, un fond de teint, un fard à paupières ou à joue, un mascara pour les cils ou les cheveux, un eye-liner, un anti-cernes, ou bien encore une composition de maquillage du corps. La composition de traitement ou de soin cosmétique peut être une composition pour le soin du visage, du cou, des mains, du corps, des ongles ou des lèvres, une composition déodorante ou encore de protection solaire.

L'invention se rapporte également à un procédé non thérapeutique de traitement ou de maquillage des matières kératiniques et/ou des muqueuses consistant à appliquer sur les matières kératiniques et/ou des muqueuses une composition telle que décrite précédemment.

L'invention a également pour objet l'utilisation d'un oligomère copolyester téréphtalique et d'un composé siliconé en dispersion aqueuse tels que définis précédemment, dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

L'invention a aussi pour objet l'utilisation d'un oligomère copolyester téréphtalique et d'un composé siliconé en dispersion aqueuse tels que définis précédemment dans une composition cosmétique ou dermatologique pour l'obtention d'un film de cette composition brillant et/ou frais et/ou confortable.

L'invention a encore pour objet l'utilisation d'une composition telle que définie précédemment pour l'obtention d'un film de cette composition brillant et/ou frais et/ou confortable et/ou ayant des propriétés de non transfert.

On va maintenant donner des exemples illustrant la présente invention sans toutefois la limiter.

**Exemple 1** **Préparation d'un oligomère copolyester téréphtalique**

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit:

- 11,47 moles de téréphtalate de diméthyle
- 2,53 moles de diméthylisophtalate-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :

- 0,5 mole d'acide isophtalique
- 2,36 moles d'acide téréphtalique
- 8 moles d'éthylène glycol

La masse réactionnelle est alors amenée à la température de 250°C en 60 minutes.

Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.

Le copolyester obtenu présente les caractéristiques structurales décrites au tableau 1 ci-après, dans lequel:

\* "% molaire des motifs diacides" correspond à la teneur, en %, de chaque diacide ou diester mise en oeuvre par rapport à l'ensemble des diacides ou diesters mis en oeuvre.

"Tp" signifie : motif téréphtalique
"Ip" signifie : motif isophtalique
"SIp" signifie : motif sulfoisophtalique

Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.

- "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.

* "%GT/$\Sigma$ motifs" correspond au % molaire des motifs de formule (I)

$$[-CO-A-CO-O-(CH_2-CH_2-O)_n-] \qquad (I)$$

où A est 1,4 phénylène et n=1
par rapport à l'ensemble des motifs de formule (I)
où A est 1,4 phénylène, sulfo 1,3 phénylène et éventuellement 1,3 phénylène et n va de 1 à 4
"%GT/$\Sigma$ motifs" se calcule par la formule suivante :

$$\%GT/\Sigma \text{ motifs} = (\% \text{ molaire de motifs Tp}) \times (\% \text{ molaire de motifs G}) / 100$$

* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| SIp | 15 |
| %GT/$\Sigma$ motifs | 46,5 |
| % molaire des motifs diols | |
| G | 56,8 |
| 2G | 30,7 |
| 3G | 10 |
| 4G | 2,5 |
| Mw | 8 000 |

### Exemple 2:

On a préparé un rouge à lèvres en forme de stick ayant la composition suivante :

- oligomère copolyester téréphtalique de l'exemple 1        20 %
- émulsion de polydiméthylsiloxane alkylé à 50 % de matières sèches (SILRES M50E de Wacker)        5 % MA
- pigments        5 %
- propylène glycol        2,5 %
- eau        qsp        100 %

On obtient un rouge à lèvres facile à appliquer et permettant l'obtention d'un film "sans transfert" total et parfaitement brillant.

**Exemple 3:**

On a préparé un rouge à lèvres en forme de stick ayant la composition suivante :

- oligomère copolyester téréphtalique de l'exemple 1        20 %
- émulsion de polydiméthyl siloxane aminée à 35 % de matières sèches (BELSIL ADM 6057E de Wacker)        10 % MA
- pigments        5 %
- propylène glycol        2,5 %
- eau        qsp        100 %

On obtient un rouge à lèvres permettant l'obtention d'un film "sans transfert" total et parfaitement brillant.

**Exemple 4:**

On a préparé un eye-liner en forme de gel ayant la composition suivante :

- oligomère copolyester téréphtalique de l'exemple 1        10 %
- émulsion de polydiméthylsiloxane phénylée à 50 % de matières sèches (MIRASIL DPDM-E de Rhône-Poulenc)        4 % MA
- pigments noirs        3 %
- propylène glycol        1,5 %
- eau        qsp        100 %

On obtient un eye-liner permettant l'obtention d'un maquillage "sans transfert" et laissant un film parfaitement brillant.

**Revendications**

**1.** Composition filmogène à application topique comprenant dans une phase aqueuse un agent gélifiant hydrophile de type polyester sulfoné, caractérisée en ce que :

i) le gélifiant hydrophile de type polyester sulfoné est un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
ii) et que la composition comprend également au moins un composé siliconé en émulsion dans la phase aqueuse,

la composition ne contenant pas 2 % en poids, par rapport au poids total de la composition, d'aminosilicone

**2.** Composition selon la revendication 1, caractérisée par le fait que l'oligomère copolyester a une masse moléculaire en poids inférieure à 15 000.

**3.** Composition selon la revendication 1 ou 2, caractérisée par le fait que l'oligomère copolyester téréphtalique a une masse moléculaire en poids allant de 5 000 à 14 000, et mieux de 8 000 à 10 000.

**4.** Composition selon l'une quelconque des revendications précédentes, caractérisée par le fait que l'oligomère copolyester téréphtalique comprend au moins 40 % en mole desdits motifs de formule (I) pour lesquels A représente un groupement 1,4-phénylène et n est égal à 1.

**5.** Composition selon l'une quelconque des revendications précédentes, caractérisée par le fait que l'oligomère copolyester téréphtalique comprend de 40 à 90 % en mole desdits motifs de formule (I) pour lesquels A représente un groupement 1,4-phénylène et n est égal à 1.

**6.** Composition selon l'une quelconque des revendications précédentes, caractérisée par le fait que l'oligomère copolyester téréphtalique comprend au moins 10 % en mole des motifs de formule (I) pour lesquels A représente un groupement sulfo-1,3-phénylène.

**7.** Composition selon l'une quelconque des revendications précédentes, caractérisée par le fait que l'oligomère copolyester téréphtalique comprend de 10 % et 25 % en mole desdits motifs de formule (I) pour lesquels A représente un groupement sulfo-1,3-phénylène.

**8.** Composition selon l'une quelconque des revendications précédentes, caractérisée par le fait que l'oligomère copolyester téréphtalique comprend jusqu'à 20 % en mole, et de préférence de 0,5 % à 5 % en mole, de motifs de formule (I) pour lesquels A représente un groupement 1,3-phénylène.

**9.** Composition selon l'une quelconque des revendications précédentes, caractérisée par le fait que l'oligomère copolyester téréphtalique est présent en une teneur allant de 0,5 % à 40 % en poids, par rapport au poids total de la composition.

**10.** Composition selon l'une quelconque des revendications précédentes sous forme solide, caractérisée par le fait que l'oligomère copolyester téréphtalique est présent en une teneur allant de 10 % à 40 % en poids, par rapport au poids total de la composition, et mieux de 15 % à 30 % en poids.

**11.** Composition selon l'une quelconque des revendications 1 à 9 sous forme de gel, caractérisée par le fait que l'oligomère copolyester téréphtalique est présent en une teneur allant de 0,5 % à 10 % en poids, par rapport au poids total de la composition, et mieux de 2 % à 5 % en poids.

**12.** Composition selon l'une des revendications précédentes, caractérisée par le fait que l'émulsion aqueuse du composé siliconé a une taille de gouttelettes allant de 10 nm à 50 μm.

**13.** Composition selon l'une quelconque des revendications précédentes, caractérisée par le fait que l'émulsion aqueuse du composé siliconé a une taille de gouttelettes allant de 10 nm à 1000 nm.

**14.** Composition selon l'une quelconque des revendications précédentes, caractérisée par le fait que le composé siliconé est un polyorganosiloxane.

**15.** Composition selon l'une quelconque des revendications précédentes, caractérisée par le fait que le composé siliconé est choisi dans le groupe formé par les huiles, les gommes, les résines, les produits pâteux, les cires de silicone.

**16.** Composition selon l'une quelconque des revendications précédentes, caractérisée par le fait que le composé siliconé est choisi dans le groupe formé par les huiles de silicones volatiles et non volatiles.

**17.** Composition selon l'une des revendications précédentes, caractérisée par le fait que le composé siliconé est choisi dans le groupe formé par les polyalkylsiloxanes, les polyarylsiloxanes, les polyalkylarylsiloxanes, les copolymères polyéthersiloxanes, organomodifiés ou non, les polysiloxanes modifiés par des groupements organofonctionnels, et leurs mélanges.

18. Composition selon l'une des revendications précédentes, dans laquelle le composé siliconé est choisi dans le groupe formé par les polydiméthylsiloxanes linéaires à groupements terminaux triméthylsilyle; les polydiméthylsiloxanes linéaires à groupements terminaux diméthylsilanol; les polydiméthylméthylphénylsiloxanes; les polydiméthyldiphénylsiloxanes.

19. Composition selon l'une des revendications précédentes, dans laquelle le composé siliconé est présent en une teneur en matière sèche allant de 0,1% à 30% en poids, par rapport au poids total de la composition, et mieux de 2% à 10% en poids.

20. Composition selon l'une quelconque des revendications précédentes, caractérisée par le fait que la composition est une composition cosmétique ou dermatologique.

21. Composition cosmétique selon l'une quelconque des revendications précédentes, caractérisée par le fait qu'elle comprend au moins un adjuvant choisi dans le groupe formé par les colorants, les pigments, les nacres, les agents anti-UV, les conservateurs, les agents épaississants, les agents plastifiants, les tensioactifs, les cires, les huiles, les parfums, les agents modificateurs de pH, les agents hydratants.

22. Composition selon l'une quelconque des revendications précédentes, caractérisée par le fait qu'elle se présente sous forme de composition de maquillage, de soin ou de traitement cosmétique des matières kératiniques et/ou muqueuses.

23. Composition selon l'une quelconque des revendications précédentes, caractérisée par le fait que la composition se présente sous la forme d'un produit de maquillage des lèvres, d'un fond de teint, d'un fard à paupières ou à joue, d'un mascara, d'un eye-liner, d'un anti-cernes, d'une composition de maquillage du corps, d'une composition pour le soin du visage, du cou, des mains, du corps, des ongles ou des lèvres, d'une composition déodorante, d'une composition de protection solaire.

24. Procédé non thérapeutique de maquillage ou de traitement des matières kératiniques et/ou des muqueuses, caractérisé par le fait que l'on applique sur les matières kératiniques et/ou des muqueuses une composition selon l'une des revendications 1 à 23.

25. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \qquad (I)$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un composé siliconé en émulsion aqueuse, dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

26. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un composé siliconé en émulsion aqueuse dans une composition cosmétique ou dermatologique pour l'obtention d'un film de cette composition brillant et/ou frais et/ou confortable.

27. Utilisation d'une composition selon l'une quelconque des revendications 1 à 23 pour l'obtention d'un film de cette composition brillant et/ou frais et/ou confortable et/ou ayant des propriétés de non transfert.

**EP 0 993 820 A1**

**Office européen des brevets**

# RAPPORT DE RECHERCHE EUROPEENNE

Numéro de la demande

EP 99 40 1799

## DOCUMENTS CONSIDERES COMME PERTINENTS

| Catégorie | Citation du document avec indication, en cas de besoin, des parties pertinentes | Revendication concernée | CLASSEMENT DE LA DEMANDE |
|---|---|---|---|
| P,X | EP 0 865 786 A (OREAL) 23 septembre 1998 (1998-09-23) * page 2, ligne 50 - page 5, ligne 19 * * page 7, ligne 4 - ligne 7 * * revendications 1-19 * | 1-27 | A61K7/027 A61K7/032 |
| P,X | FR 2 760 636 A (OREAL) 18 septembre 1998 (1998-09-18) * revendications 1-17; exemple 5 * | 1-27 | |
| D,P, X | EP 0 864 319 A (OREAL) 16 septembre 1998 (1998-09-16) * page 5, ligne 40 - ligne 44 * * exemple 5 * * revendications 1-14 * | 1-27 | |
| A | FR 2 742 986 A (RHONE POULENC CHIMIE) 4 juillet 1997 (1997-07-04) | | |
| A | US 5 389 363 A (SNYDER FLORENCE ET AL) 14 février 1995 (1995-02-14) | | DOMAINES TECHNIQUES RECHERCHES |
| A | EP 0 792 636 A (OREAL) 3 septembre 1997 (1997-09-03) | | A61K |

Le présent rapport a été établi pour toutes les revendications

| Lieu de la recherche | Date d'achèvement de la recherche | Examinateur |
|---|---|---|
| LA HAYE | 27 août 1999 | Stienon, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

14

**ANNEXE AU RAPPORT DE RECHERCHE EUROPEENNE
RELATIF A LA DEMANDE DE BREVET EUROPEEN NO.**

EP 99 40 1799

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-08-1999

| Document brevet cité au rapport de recherche | | Date de publication | Membre(s) de la famille de brevet(s) | | Date de publication |
|---|---|---|---|---|---|
| EP 0865786 | A | 23-09-1998 | FR 2760634 A<br>CA 2230131 A<br>JP 2865656 B<br>JP 10306039 A | | 18-09-1998<br>14-09-1998<br>08-03-1999<br>17-11-1998 |
| FR 2760636 | A | 18-09-1998 | WO 9841184 A | | 24-09-1998 |
| EP 0864319 | A | 16-09-1998 | FR 2760642 A<br>CA 2230137 A<br>JP 10259120 A | | 18-09-1998<br>14-09-1998<br>29-09-1998 |
| FR 2742986 | A | 04-07-1997 | AU 1381197 A<br>CN 1207668 A<br>EP 0869767 A<br>WO 9724104 A<br>JP 11501673 T | | 28-07-1997<br>10-02-1999<br>14-10-1998<br>10-07-1997<br>09-02-1999 |
| US 5389363 | A | 14-02-1995 | AUCUN | | |
| EP 0792636 | A | 03-09-1997 | FR 2745495 A<br>BR 9700306 A<br>CA 2198769 A<br>CN 1168791 A<br>DE 69700002 D<br>DE 69700002 T<br>ES 2119579 T<br>US 5866111 A | | 05-09-1997<br>27-10-1998<br>29-08-1997<br>31-12-1997<br>07-05-1998<br>30-07-1998<br>01-10-1998<br>02-02-1999 |

EPO FORM P0460

Pour tout renseignement concernant cette annexe : voir Journal Officiel de l'Office européen des brevets, No.12/82